(19) Europäisches Patentamt

European Patent Office

Office européen des brevets

(11) EP 4 681 626 A1

(12) EUROPEAN PATENT APPLICATION
published in accordance with Art. 153(4) EPC

(43) Date of publication:
21.01.2026 Bulletin 2026/04

(21) Application number: 24769666.9

(22) Date of filing: 25.01.2024

(51) International Patent Classification (IPC):
A61B 5/05 (2021.01)    A61B 5/103 (2006.01)

(52) Cooperative Patent Classification (CPC):
A61B 5/05; A61B 5/103

(86) International application number:
PCT/CN2024/074075

(87) International publication number:
WO 2024/187970 (19.09.2024 Gazette 2024/38)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 16.03.2023 CN 202310253897

(71) Applicant: Medlander Medical Technology Inc.
Nanjing, Jiangsu 210012 (CN)

(72) Inventors:
• MA, Zhangyin
  Nanjing, Jiangsu 210012 (CN)
• LV, Zhiwei
  Nanjing, Jiangsu 210012 (CN)
• BI, Zhengyang
  Nanjing, Jiangsu 210012 (CN)
• LI, Miao
  Nanjing, Jiangsu 210012 (CN)

(74) Representative: Michalski Hüttermann & Partner
Patentanwälte mbB
Kaistraße 16A
40221 Düsseldorf (DE)

(54) METHOD AND SYSTEM FOR DETECTING PELVIC FLOOR MUSCLE MOVEMENT BASED ON MILLIMETER WAVE RADAR

(57) The present application discloses a method and system for detecting pelvic floor muscle movement based on millimeter-wave radar, applicable to the field of medical technology. The method comprises: performing range FFT on the intermediate frequency signal to obtain a range FFT result, wherein the range FFT result includes multiple sets of data points corresponding one-to-one to multiple range-bins; selecting data points corresponding to target range-bin from the range FFT result as information to be processed, wherein the target range-bin matches the range between the pelvic floor muscles and the millimeter-wave radar; demodulating the information to be processed to obtain phase information; and converting the phase information into pelvic floor muscle displacement information. This application proposes a novel non-contact method for detecting pelvic floor muscles based on millimeter-wave radar. This non-contact method can avoid the discomfort caused to patients by physical contact during detection, making it easy to promote.

200

acquiring an intermediate frequency signal
210

performing range FFT on the intermediate frequency signal to obtain a range FFT result
220

selecting data points corresponding to target range-bin from the range FFT result as the information to be processed
240

demodulating the information to be processed to obtain phase information
250

converting the phase information into pelvic floor muscle displacement information
260

identifying pelvic floor muscle movement pattern based on the pelvic floor muscle displacement information
280

real-time displaying the pelvic floor muscle displacement information and/or the identified pelvic floor muscle movement pattern
290

FIG.2

**Description**

**CROSS REFERENCE TO RELATED APPLICATIONS**

This application claims priority of Chinese invention patent application 202310253897.8 filed on March 16, 2023, the entire contents of which are hereby incorporated by reference.

**TECHNICAL FIELD**

This application relates to the field of medical technology, in particularly to a method and system for detecting pelvic floor muscle movement based on millimeter wave radar.

**BACKGROUND OF THE INVENTION**

Millimeter-wave technology features short wavelengths, wide bandwidth, high resolution, narrow antenna beams, strong penetration capabilities, and excellent interference resistance. Millimeter-wave sensors enable high-resolution, high-sensitivity, and high-accuracy target perception and detection, therefore widely applied in fields such as information security, healthcare, autonomous driving, and emergency search and rescue, with significant practical value.
The pelvic floor muscles refer to the muscle group that closes the pelvic floor, which acts like a "suspension net," holding organs such as the urethra, bladder, vagina, uterus, and rectum in place to maintain their normal positions and perform their functions. Taking women as an example, pelvic floor muscle dysfunction such as vaginal relaxation and stress urinary incontinence caused by pregnancy, childbirth, and aging require exercise the pelvic floor muscles to prevent pelvic floor muscle diseases and address pelvic floor muscle functional disorders. The existing pelvic floor muscle detection method is typically to detect pelvic floor muscle vitality by measuring the contraction pressure of the vaginal wall. This method requires professional personnel to operate, and during the testing process, the operator needs to make physical contact with the patient, which may cause discomfort to the patient. Contact-based pelvic floor muscle testing method has been rejected by many people, making it difficult to promote on a large scale and posing challenges to pelvic floor muscle detection.
Currently, there is a need for a non-contact pelvic floor muscle detection method based on millimeter-wave radar.

**SUMMARY OF THE INVENTION**

The objective of this application is to provide a non-contact method for detecting pelvic floor muscles based on millimeter-wave radar.
The first aspect of the present application provides a method for detecting pelvic floor muscle movement based on millimeter-wave radar, comprising: acquiring an intermediate frequency signal; performing range FFT on the intermediate frequency signal to obtain a range FFT result, wherein the range FFT result includes multiple sets of data points corresponding one-to-one to multiple range-bins; selecting data points corresponding to target range-bin from the range FFT result as the information to be processed, wherein the target range-bin matches the range between the pelvic floor muscles and the millimeter-wave radar; demodulating the information to be processed to obtain phase information; and converting the phase information into pelvic floor muscle displacement information.
The second aspect of the present application provides a system for detecting pelvic floor muscle movement based on millimeter-wave radar, comprising: an acquisition module for acquiring intermediate frequency signal; an FFT module for performing range FFT on the intermediate frequency signal to obtain the range FFT result, wherein the range FFT result includes multiple sets of data points corresponding one-to-one to multiple range-bins; a selection module for selecting data points corresponding to target range-bin from the range FFT result as the information to be processed, wherein the target range-bin matches the range between the pelvic floor muscles and the millimeter-wave radar; a demodulation module for demodulating the information to be processed to obtain phase information; and a conversion module for converting the phase information into pelvic floor muscle displacement information.
The third aspect of the present application provides a device for detecting pelvic floor muscle movement, comprising a processor and a memory, wherein the memory stores a computer program, and when the processor executes the computer program, performs the following operations: acquiring intermediate frequency signal; performing range FFT on the intermediate frequency signal to obtain a range FFT result, wherein the range FFT result includes multiple sets of data points corresponding one-to-one to multiple range-bins; selecting data points corresponding to the target range-bin from the range FFT result as information to be processed, wherein the target range-bin matches the range between the pelvic floor muscles and the millimeter-wave radar; demodulating the information to be processed to obtain phase information; and converting the phase information into pelvic floor muscle displacement information.
The fourth aspect of the present application provides a computer-readable storage medium, wherein the computer storage medium stores a computer program, when the computer program is executed on a computer, the computer performs the following operations: acquiring an intermediate frequency signal; performing range FFT on the intermediate frequency signal to obtain a range FFT result, wherein the range FFT result includes multiple sets of data points corresponding one-to-one to multiple range-bins; selecting data points corresponding to target range-bin from the range FFT result as information to be processed, wherein the target range-bin matches the range between the pelvic floor muscles and the millimeter-wave radar; demodulating the information to be processed to obtain phase information; and converting the phase information into pelvic floor muscle displacement information.

This application proposes a novel non-contact method for detecting the pelvic floor muscles based on millimeter-wave radar. This non-contact method avoids the discomfort caused to patients by physical contact during detection and is easy to implement.

## BRIEF DESCRIPTION OF THE DRAWINGS

The accompanying drawings are incorporated into and form part of the specification, illustrating embodiments of the present application, and are used together with the specification to explain the principles of the present application, wherein:

FIG.1 is an exemplary block diagram of the pelvic floor muscle movement detection system based on millimeter-wave radar according to some embodiments of the present application;
FIG.2 is an exemplary flowchart illustrating the pelvic floor muscle movement detection method based on millimeter-wave radar according to some embodiments of the present application;
FIG.3 is a structural schematic diagram of the millimeter-wave radar system according to some embodiments of the present application;
FIG.4 is a schematic diagram of the pelvic floor muscle movement detection process according to some embodiments of the present application;
FIG.5 is an exemplary block diagram of the pelvic floor muscle movement detection device according to some embodiments of the present application.

## DETAILED DESCRIPTION OF THE INVENTION

In order to have a clearer understanding of the above objectives, features, and advantages of this application, the following sections will provide a further description of the embodiments of this application. It should be noted that, where not conflicting, the embodiments of this application and the features within them may be combined with each other.

The following description includes many specific details to facilitate a thorough understanding of the present application; however, the present application may be implemented in other ways that differ from those described herein. Obviously, the embodiments described in the specification are only a part of the embodiments of the present application, not all of them.

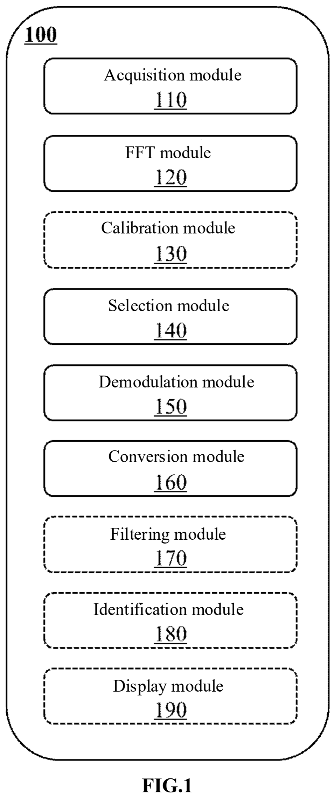

FIG.1 is an exemplary block diagram of pelvic floor muscle movement detection system based on millimeter-wave radar according to some embodiments of the present application. As shown in FIG.1, the system 100 includes an acquisition module 110, an FFT (Fast Fourier Transform) module 120, a selection module 140, a demodulation module 150, and a conversion module 160.

The acquisition module 110 is used to acquire an intermediate frequency signal.

The FFT module 120 is used to perform range FFT on the intermediate frequency signal to obtain a range FFT result, which includes multiple sets of data points corresponding one-to-one to multiple range-bins.

The selection module 140 is used to select data points corresponding to the target range-bin from the range FFT result as information to be processed, where the target range-bin matches the range between the pelvic floor muscles and the millimeter-wave radar.

The demodulation module 150 is used to demodulate the information to be processed to obtain phase information.

The conversion module 160 is used to convert the phase information into pelvic floor muscle displacement information.

In some embodiments, the system 100 further includes a calibration module 130, which is used to: performing circular fit on the range FFT result to obtain a fitting result, wherein the fitting result includes the position of the center of the circle; determining the DC offset based on the fitting result; calibrating the range FFT result based on the DC offset to obtain a calibrated range FFT result. Accordingly, selecting the data points corresponding to the target range-bin from the range FFT result as the information to be processed includes: selecting the data points corresponding to the target range-bin from the calibrated range FFT result as the information to be processed.

In some embodiments, the system 100 further includes an identification module 180, which is used to identify pelvic floor muscle movement pattern based on the pelvic floor muscle displacement information.

In some embodiments, the system 100 further includes a filtering module 170, which is used to: filter the pelvic floor muscle displacement information to obtain the filtered pelvic floor muscle displacement information. Accordingly, identifying pelvic floor muscle movement pattern based on the pelvic floor muscle displacement information includes: identifying pelvic floor muscle movement pattern based on the filtered pelvic floor muscle displacement information.

In some embodiments, the pelvic floor muscle displacement information includes displacements at multiple time points, and the pelvic floor muscle movement pattern includes contraction, relaxation, contracting, and relaxing. Accordingly, identifying the pelvic floor muscle movement pattern based on the pelvic floor muscle displacement information includes: calculating the displacement mean and displacement standard deviation corresponding to each time point based on the pelvic floor muscle displacement information according to a predefined sliding window length; and determining the pelvic floor muscle movement pattern corresponding to each time point based on the displacement mean and displacement standard deviation corresponding to each time point.

In some embodiments, the system 100 further includes a display module 190, which is used to display the identified pelvic floor muscle movement pattern in real time.

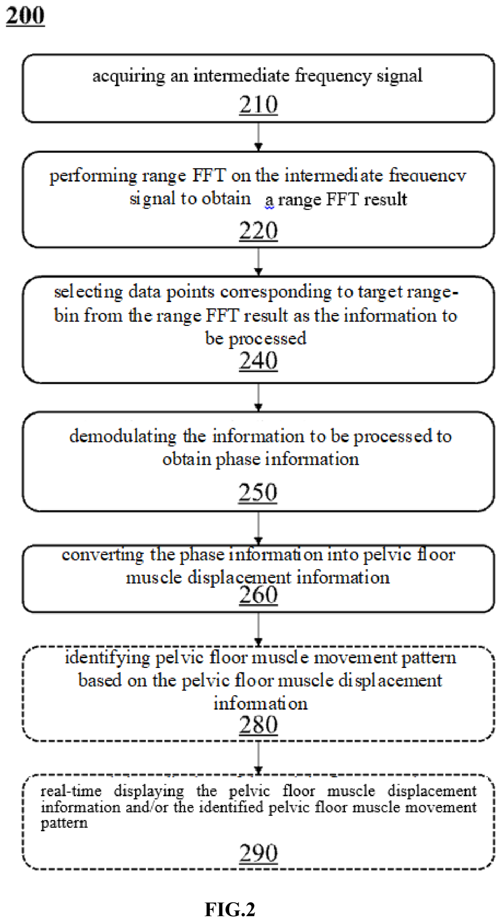

For more details about the system 100 and its modules, refer to FIG. 2 and its related descriptions.

FIG. 2 is an exemplary flowchart of a pelvic floor muscle movement detection method based on millimeter-wave radar according to some embodiments of the present application. Flowchart 200 can be executed by at least one processor. In some embodiments, flowchart 200 may be executed by the pelvic floor muscle movement detection system 100 shown in FIG. 1. As shown in FIG.2, flowchart 200 includes the following steps.

Step 210: Acquiring an intermediate frequency signal.

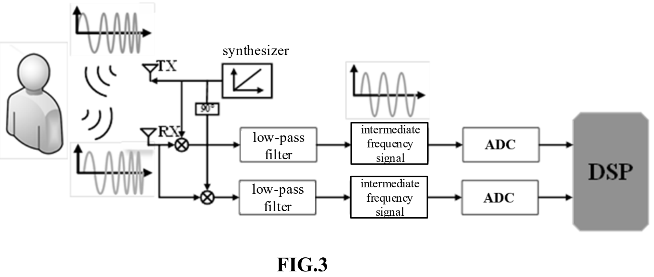

The intermediate frequency signal here may refer to the intermediate frequency signal obtained through A/D sampling. A/D sampling refers to converting a continuous-time signal (i.e., analog signal) into a discrete-time signal (i.e., digital signal) at a certain sampling rate. Taking FMCW (Frequency Modulated Continuous Wave) radar as an example, refer to FIG.3, the synthesizer generates a linearly frequency-modulated pulse signal (also known as a chirp signal), the transmit antenna (denoted as TX) is responsible for transmitting the chirp signal, and the receive antenna is responsible for capturing the signal reflected by objects along the radar transmission path (i.e., echo signal). The mixer combines (also known as mixes) the radar transmitted signal and the radar echo signal. The combined (mixed) signal then passes through a low-pass filter, and the output signal from the low-pass filter is referred to as the intermediate frequency (IF) signal. The IF signal is sent to the ADC (Analog-to-Digital Converter). Finally, the DSP processes the IF signal that has been sampled by the A/D converter.

Step 220: Performing range FFT on the intermediate frequency signal to obtain a range FFT result.

The range FFT result includes multiple sets of data points corresponding one-to-one to multiple range-bins. In the radar field of view, when two targets are located at the same azimuth angle but at different ranges from the radar, the minimum range at which they can be distinguished is the radar's range resolution. The radar's detection range is divided into multiple units (i.e., multiple sub-ranges) based on its range resolution. Each unit (sub-range) corresponds to a range-bin (also known as rang-unit). The range FFT result can be represented in matrix form, where each column (or row) corresponds to a range bin, and each row (or column) corresponds to a linear frequency-modulated signal (chirp signal). The range FFT result is essentially I/Q signal, which can be represented by amplitude and phase in polar coordinates, or by the real part (in-phase component, denoted as I) and imaginary part (out-of-phase component, denoted as Q) in Cartesian coordinates.

Step 240: Selecting the data points corresponding to the target range-bin from the range FFT result as the information to be processed.

The target range-bin matches the range from the pelvic floor muscles to the millimeter-wave radar. That is, the range from the pelvic floor muscles to the millimeter-wave radar falls within the target range-bin. The target range-bin may include at least one range-bin. In actual application, the patient may remain seated, with the millimeter-wave radar located below the patient, such as placed under a chair. It should be noted that although the patient is in a seated position, the patient can control the movement of their pelvic floor muscles to cooperate with the detection. Sitting still helps eliminate interference from irrelevant signal (e.g., body movement signal) during signal processing.

In some embodiments, the target range-bin can be determined through prior measurement. In some embodiments, the target range-bin can be determined by analyzing the range FFT result. Specifically, the data points corresponding to the target range-bin (e.g., pelvic floor muscles) appear as peak point in the range FFT result. A peak point refers to the data point with the maximum (local) amplitude. In some embodiments, the target range-bin can first be roughly determined through preliminary measurements, and then precisely determined by identifying the peak points.

Step 250: Demodulating the information to be processed to obtain phase information.

As an example, the demodulation method used in step 250 includes the inverse tangent demodulation algorithm.

Step 260: Converting the phase information into pelvic floor muscle displacement information.

There is a mapping relationship between the phase information and the pelvic floor muscle displacement information.

Taking FMCW radar as an example, the transmitted signal (i.e., the first chirp signal) within the first scanning cycle can be expressed as:

$$x_{tx}(t) = A_{TX}\cos(\mu(t)) = A_{TX}\cos\left(2\pi\left(f_c t + \frac{\alpha t^2}{2}\right) + \varphi_0\right) \qquad (1).$$

Wherein, $x_{tx}(t)$ denotes the transmitted signal, t denotes time, $A_{TX}$ denotes amplitude, $f_c$ denotes the initial frequency (frequency at t=0), $\alpha$ denotes the rate of change of frequency with time, and $\varphi_0$ denotes the initial phase (phase at t=0).

Assuming that the displacement of the pelvic floor muscle is $R(t)$, and $d_0$ is the initial distance (distance at t=0) between the pelvic floor muscle and the millimeter-wave radar. Generally, the distance between the pelvic floor muscle and the millimeter-wave radar is $x(t) = R(t) + d_0$, so the time delay between the echo signal and the transmitted signal is $\tau = 2x(t)/c$, wherein $c$ is the speed of light.

Correspondingly, the received signal (i.e., the echo signal) can be expressed as:

$$x_{rx}(t) = A_{RX}\cos(\mu(t-\tau)) = A_{RX}\cos\left(2\pi\left(f_c(t-\tau) + \frac{\alpha(t-\tau)^2}{2}\right) + \varphi_0\right) \qquad (2).$$

The output signal of the mixer can be expressed as:

$$x_m(t) = x_{tx}(t)x_{rx}(t) \qquad (3).$$

Equation (3) can be converted using trigonometric formulas $\cos(\alpha)\cos(\beta) = (\cos(\alpha + \beta) + \cos(\alpha - \beta))/2$. The output signal $x_m(t)$ of the mixer is filtered by a low-pass filter, and the intermediate frequency signal obtained (also denoted as $x_m(t)$) can be expressed as:

$$x_m(t) = \frac{A_{TX}A_{RX}}{2}\cos\left(2\pi\left(f_c t + \frac{\alpha t^2}{2} - f_c(t-\tau) - \frac{\alpha(t-\tau)^2}{2}\right)\right) \qquad (4).$$

Simplifying equation (4) yields:

$$x_m(t) = \frac{A_{TX}A_{RX}}{2}\cos\left(2\pi\left(f_c\tau + \alpha\tau t - \frac{\alpha\tau^2}{2}\right)\right) \qquad (5).$$

Performing an orthogonal transformation on equation (5) yields the complex representation of the difference frequency signal (i.e., the intermediate frequency signal):

$$S_{IF}(t) = A_{TX} A_{RX} \exp\left( j\left( 2\pi\alpha\tau t + 2\pi f_c \tau + \pi\alpha\tau^2 \right) \right)$$

$$\approx A_{TX} A_{RX} \exp\left( j\left( 2\pi\alpha\tau t + 2\pi f_c \tau \right) \right)$$

$$= A_{TX} A_{RX} \exp\left( j(2\pi \underbrace{\frac{2Bd_0}{cT_c}}_{f_b} t + \underbrace{\frac{4\pi\left( R(t) + d_0 \right)}{\lambda}}_{\psi(t)}) \right)$$

$$= A_{TX} A_{RX} \exp\left( j\left( 2\pi f_b t + \psi(t) \right) \right) \tag{6}.$$

Wherein $\tau = 2(R(t) + d_0)/c$, because $\pi\alpha\tau^2$ is so small in practical situations that it can be ignored. From equation (6), it can be seen that there is a linear relationship between the phase $\psi(t)$ and the pelvic floor muscle displacement R(t).

Since the speed of pelvic floor muscle movement is far below $10^3$ m/s, the pelvic floor muscle displacement R(t) is very small. For example, when the duration of a single chirp signal is 1500 $\mu$s, the speed of pelvic floor muscle movement does not exceed 1 mm/chirp. When R(t) is sufficiently small, R(t) is approximately constant over the duration of a single chirp signal. To obtain the pelvic floor muscle displacement R(t), multiple chirp signals must be sequentially transmitted, which is equivalent to sampling R(t). Assuming that L chirp signals are sent per frame, and the sampling interval of R(t) is the frame period (i.e., the duration of one frame, denoted as T), the time delay will become:

$$\tau = \frac{2\left[ d_0 + R\left( lT + t \right) \right]}{c} \tag{7}.$$

The sampled intermediate frequency signal can be expressed as:

$$S_{IF}\left( lT + nT_s \right) = A_T A_R \exp\left( j\left( 2\pi f_b nT_s + \psi_l \right) \right) \tag{8}$$

$$f_b = \frac{2Bd_0}{cT} \tag{9}$$

$$\psi_l = \frac{4\pi\left( R\left( lT \right) + d_0 \right)}{\lambda} \tag{10}$$

Wherein, T denotes the slow-time sampling period, $l$ denotes the frame number, $T_s$ denotes the fast-time sampling period, and n denotes the chirp signal number. Thus, the sampled intermediate-frequency signal is a two-dimensional function of the fast-sampling dimension (corresponding to n) and the slow-time dimension (corresponding to 1). Combining the above equations, the system can extract frequency $f_b$ and phase $\psi_l$ information from the sampled intermediate frequency signal to estimate the distance $d_0$ and displacement R(t) of the pelvic floor muscles. As mentioned earlier, the distance $d_0$ and displacement R(t) can be obtained through FFT and demodulation.

In some embodiments, the calibration module 130 may perform I/Q signal calibration on the range FFT result. Specifically, the calibration module 130 can perform a circular fit on the range FFT result to obtain the fitting result, which includes the position of the center of the circle. After obtaining the fitting result, the calibration module 130 can determine the DC offset based on the fitting result and calibrate the range FFT result based on the DC offset to obtain the calibrated range FFT result. Ideally, the distribution of the I/Q signal (range FFT result) is a circle centered at the origin of the coordinate system, and the DC offset is the offset of the fitted center relative to the origin of the coordinate system. Calibration can eliminate the DC offset in the I/Q signal, i.e., moves the fitted center to the origin. Furthermore, the selection module 140 can select the data points corresponding to the target range-bin from the calibrated range FFT result as the information to be processed. Calibrating the range FFT result helps ensure that the demodulated phase information has a certain linear relationship with the pelvic floor muscle displacement information, thereby helping to ensure the detection accuracy of pelvic floor muscle movement.

In some embodiments, the flowchart 200 further includes a pattern recognition step 280.

Step 280, identifying the pelvic floor muscle movement pattern based on the pelvic floor muscle displacement information.

As mentioned earlier, when the sampling interval of pelvic floor muscle displacement is the frame period, one displacement sample point can be obtained within each frame. That is, the pelvic floor muscle displacement information can include displacement at multiple time points (e.g., each frame is considered a time point). The identification module 180 can identify the pelvic floor muscle movement pattern corresponding to each time point based on the pelvic floor muscle displacement information. For example, as will be described below, the identification module 180 can identify the pelvic floor muscle movement pattern corresponding to an observation window based on the mean and standard deviation of multiple data points within the single observation window. For example, the identification module 180 may utilize a trained machine learning model to predict the pelvic floor muscle movement pattern, wherein the machine learning model includes but is not limited to a neural network.

In some embodiments, the pelvic floor muscle displacement information includes displacement at multiple time points, and the pelvic floor muscle movement pattern includes contraction (maintaining a contracted state), relaxation (maintaining a relaxed state), contracting (the action of transitioning from a relaxed state to a contracted state), and relaxing (the action of transitioning from a contracted state to a relaxed state). The identification module 180 may calculate the displacement mean and displacement standard deviation corresponding to each time point based on the pelvic floor muscle displacement information according to a predefined sliding window length. Each time point (e.g., each frame) corresponds to a fixed-length (e.g., a fixed number of data points or a fixed duration) observation window, For each time point (window), all data points within the window centered at that time point (e.g., the data point and the 20 data points surrounding it, or all data points within 0.1 seconds) . Subsequently, the identification module 180 can determine the pelvic floor muscle movement pattern corresponding to each time point based on the displacement mean and displacement standard deviation corresponding to each time point. As an example, for any time point: if its corresponding mean does not exceed 0.3 mm and its corresponding standard deviation does not exceed 0.2 mm, the pelvic floor muscle movement pattern corresponding to that time point is considered to be relaxed; if its corresponding mean exceeds 0.3 mm and its corresponding standard deviation does not exceed 0.2 mm, the pelvic floor muscle movement pattern corresponding to that time point is considered to be contracted; if the corresponding standard deviation exceeds 0.2 mm, the pelvic floor muscle movement pattern corresponding to that time point is considered to be either relaxing or contracting. It can be understood that in the third case, whether the pelvic floor muscle movement pattern corresponding to that time point is relaxing or contracting depends on the previously determined pelvic floor muscle movement pattern corresponding to the previous time point. In other words, in the third case, if the previous time point corresponds to relaxation, then the current time point corresponds to contracting. Similarly, if the previous time point corresponds to contraction, then the current time point corresponds to relaxing. In practical applications, the thresholds mentioned in the above examples can be appropriately adjusted, and these thresholds can be determined based on human experience and/or experimental results.

In some embodiments, the filtering module 170 can filter the pelvic floor muscle displacement information. Subsequently, the identification module 180 can identify the pelvic floor muscle movement pattern based on the filtered pelvic floor muscle displacement information. Since the pelvic floor muscle is a relatively complex detection environment, the accuracy of the demodulated pelvic floor muscle displacement information needs to be improved. Therefore, filtering can effectively ensure the detection accuracy of pelvic floor muscle movement.

In some embodiments, the filtering module 170 may perform mean filtering on the pelvic floor muscle displacement information to obtain the mean-filtered pelvic floor muscle displacement information. As an example, mean filtering may use a template with a length of 8 data points. After filtering, the value of each data point is replaced by the average value of all data points within the template centered on that data point (including the data point itself). Then, the filtering module 170 can perform Kalman filtering on the mean-filtered pelvic floor muscle displacement information to obtain Kalman-filtered pelvic floor muscle displacement information. Subsequently, the identification module can identify pelvic floor muscle movement pattern based on the Kalman-filtered pelvic floor muscle displacement information. On the one hand, mean filtering helps achieve real-time noise removal and obtain high-precision pelvic floor muscle displacement information. On the other hand, using Kalman filtering (e.g., traditional or improved Kalman filtering algorithms) helps to remove the effects of respiratory signal and other noise (e.g., environmental noise) in real time, and helps to address baseline drift issues caused by human respiratory movements, body movements, and the nonlinearity of radar devices (baseline drift issues are particularly severe in ultra-close-range detection of complex environments).

In some embodiments, flowchart 200 further includes a real-time display step 290.

Step 290, real-time displaying the pelvic floor muscle displacement information and/or the identified pelvic floor muscle movement pattern.

Display module 190 may directly display the identified pelvic floor muscle movement pattern. Alternatively, display module 190 may display the pelvic floor muscle displacement information, with professionals determining the pelvic floor muscle movement pattern based on the pelvic floor muscle displacement information. It should be noted that the displayed pelvic floor muscle displacement information may refer to filtered (e.g., mean filtering and Kalman filtering) pelvic floor muscle displacement information. Of course, the display module 190 may simultaneously display the pelvic floor muscle displacement information and the identified pelvic floor muscle movement pattern. In some embodiments, the real-time display content may also include other information, such as radar echo signal.

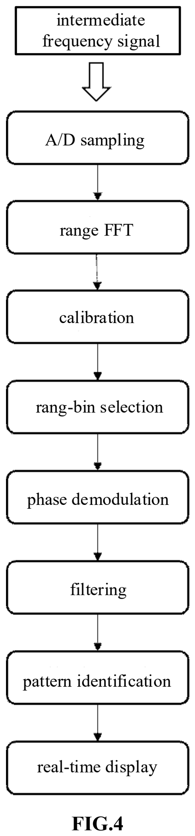

FIG.4 is a schematic diagram of the pelvic floor muscle movement detection process according to some embodiments of the present application. As shown in FIG. 4, after acquiring the intermediate frequency signal, the system 100 performs A/D sampling on the intermediate frequency signal. Then, the system 100 performs a range FFT on the A/D-sampled intermediate frequency signal and calibrates the range FFT results. Next, the system 100 selects data points corresponding to the target range-bin from the calibrated range FFT result as the information to be processed, and performs phase demodulation on the information to be processed to obtain phase information. Subsequently, the system 100 filters the pelvic floor muscle displacement information mapped from the phase information. Finally, the system 100 identifies the pelvic floor muscle movement pattern based on the filtered pelvic floor muscle displacement information and displays the pelvic floor muscle displacement information and the identified pelvic floor muscle movement pattern in real time.

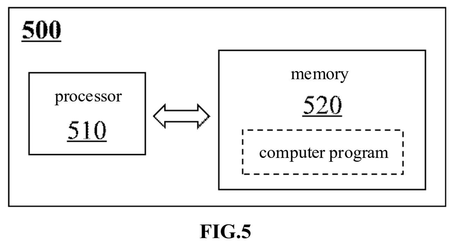

FIG. 5 is an exemplary block diagram of a pelvic floor muscle movement detection device according to some embodiments of the present application. As shown in FIG. 5, the pelvic floor muscle movement detection device 500 (hereinafter referred to as the detection device 500) includes a processor 510 and a memory 520. The memory 520 stores a computer program. When the processor 510 executes the computer program, it implements the millimeter-wave radar-based pelvic floor muscle movement detection method provided by the embodiments of the present application. Further details of the method can be found elsewhere in this specification, such as in FIG. 2 and its related descriptions. The pelvic floor muscle movement detection system 100 shown in FIG. 1 can be implemented in the detection device 500 (e.g., in a DSP).

In some embodiments, at least a portion of the detection device 500 may be integrated into a radar device. For example, the processor 510 and memory 520 in the detection device 500 may be integrated into the radar device in the form of a DSP (Digital Signal Processor). It should be understood that the combination of the detection device 510 and the radar device may be regarded as a radar system.

In some embodiments, the detection device 500 may include a display component (not shown) for real-time display of the identified pelvic floor muscle movement pattern. Of course, the detection device 500 may also achieve real-time display of pelvic floor muscle movement pattern through an external display device.

It should be noted that in this document, the terms "including" ,"comprising" or any other variations thereof are intended to encompass non-exclusive inclusion, such that a process, method, article, or device that includes a series of elements not only includes those elements but also includes other elements not explicitly listed, or elements inherent to the process, method, article, or device. Without further limitation, elements defined by the phrase "including a..." do not exclude the presence of additional identical elements in the process, method, article, or device that includes the said elements.

The above description is merely an embodiment of the present application, and the embodiments enable those skilled in the art to understand and implement the present application. Various modifications to the embodiments described herein will be apparent to those skilled in the art, and the general principles defined herein may be applied in other embodiments without departing from the spirit or scope of the present application. Therefore, the present application will not be limited to the embodiments described herein, but rather to the broadest scope consistent with the principles and features disclosed herein.

**Claims**

1. A method for detecting pelvic floor muscle movement based on millimeter-wave radar, comprising:

   acquiring an intermediate frequency signal;
   performing range fast Fourier transform (FFT) on the intermediate frequency signal to obtain a range FFT result, wherein the range FFT result includes multiple sets of data points corresponding one-to-one to multiple range-bins;
   selecting data points corresponding to target range-bin from the range FFT result as the information to be processed, wherein the target range-bin matches the range between the pelvic floor muscles and the millimeter-wave radar;
   demodulating the information to be processed to obtain phase information;
   converting the phase information into pelvic floor muscle displacement information.

2. The method according to claim 1, further comprising:

performing circular fit on the range FFT result to obtain a fitting result, which includes the position of the center of the circle;

determining the DC offset based on the fitting result;

calibrating the range FFT result based on the DC offset to obtain a calibrated range FFT result;

selecting data points corresponding to the target range-bin from the range FFT result as information to be processed, comprising:

selecting the data points corresponding to the target range-bin from the calibrated range FFT result as the information to be processed.

3. The method according to claim 1, further comprising: identifying the pelvic floor muscle movement pattern based on the pelvic floor muscle displacement information.

4. The method according to claim 3, further comprising:

performing mean filtering on the pelvic floor muscle displacement information to obtain mean-filtered pelvic floor muscle displacement information;

performing Kalman filtering on the mean-filtered pelvic floor muscle displacement information to obtain Kalman-filtered pelvic floor muscle displacement information,

wherein identifying the pelvic floor muscle movement pattern based on the pelvic floor muscle displacement information, comprising:

identifying the pelvic floor muscle movement pattern based on the Kalman-filtered pelvic floor muscle displacement information.

5. The method according to claim 3, wherein the pelvic floor muscle displacement information includes displacement at multiple time points, and the pelvic floor muscle movement pattern includes contraction, relaxation, contracting, and relaxing;

wherein identifying the pelvic floor muscle movement pattern based on the pelvic floor muscle displacement information, comprising:

calculating the displacement mean and displacement standard deviation corresponding to each time point based on the pelvic floor muscle displacement information according to a predefined sliding window length;

determining the pelvic floor muscle movement pattern corresponding to each time point based on the displacement mean and displacement standard deviation corresponding to each time point.

6. The method according to claim 3, further comprising: real-time displaying the pelvic floor muscle displacement information and/or the identified pelvic floor muscle movement pattern.

7. A system for detecting pelvic floor muscle movement based on millimeter-wave rada, comprising:

an acquisition module for acquiring an intermediate frequency signal;

a fast Fourier transform (FFT) module for performing range FFT on the intermediate frequency signal to obtain a range FFT result, wherein the range FFT result includes multiple sets of data points corresponding one-to-one to multiple range-bins;

a selection module for selecting data points corresponding to target range-bin from the range FFT result as the information to be processed, wherein the target range-bin matches the range between the pelvic floor muscles and the millimeter-wave radar;

a demodulation module for demodulating the information to be processed to obtain phase information;

a conversion module for converting the phase information into pelvic floor muscle displacement information.

8. The system according to claim 7, further comprising a calibration module for:

performing circular fit on the range FFT result to obtain a fitting result, which includes the position of the center of the circle;

determining the DC offset based on the fitting result;

calibrating the range FFT result based on the DC offset to obtain a calibrated range FFT result;

selecting the data points corresponding to the target range-bin from the range FFT result as the information to be processed, comprising:

selecting the data points corresponding to the target range-bin from the calibrated range FFT result as the information to be processed.

9. The system according to claim 7, further comprising: an identification module for identifying pelvic floor muscle movement pattern based on the pelvic floor muscle displacement information.

10. The system according to claim 9, further comprising a filtering module for:

performing mean filtering on the pelvic floor muscle displacement information to obtain mean-filtered pelvic floor muscle displacement information;
performing Kalman filtering on the mean-filtered pelvic floor muscle displacement information to obtain Kalman-filtered pelvic floor muscle displacement information;
wherein identifying the pelvic floor muscle movement pattern based on the pelvic floor muscle displacement information, comprising:
identifying the pelvic floor muscle movement pattern based on the Kalman-filtered pelvic floor muscle displacement information.

11. The system according to claim 9, wherein the pelvic floor muscle displacement information includes displacement at multiple time points, and the pelvic floor muscle movement pattern includes contraction, relaxation, contracting, and relaxing;
wherein identifying the pelvic floor muscle movement pattern based on the pelvic floor muscle displacement information, comprising:

calculating the displacement mean and displacement standard deviation corresponding to each time point based on the pelvic floor muscle displacement information according to a predefined sliding window length;
determining the pelvic floor muscle movement pattern corresponding to each time point based on the displacement mean and displacement standard deviation corresponding to each time point.

12. The system according to claim 9, further comprising a display module for real-time displaying the pelvic floor muscle displacement information and/or the identified pelvic floor muscle movement pattern.

13. A device for detecting pelvic floor muscle movement, comprising a processor and a memory, wherein the memory stores a computer program, and when the processor executes the computer program, it performs the following operations:

acquiring an intermediate frequency signal;
performing range fast Fourier transform (FFT) on the intermediate frequency signal to obtain a range FFT result, which includes multiple sets of data points corresponding one-to-one to multiple range-bins;
selecting data points corresponding to the target range-bin from the range FFT result as information to be processed, wherein the target range-bin matches the range between the pelvic floor muscles and the millimeter-wave radar;
demodulating the information to be processed to obtain phase information;
converting the phase information into pelvic floor muscle displacement information.

14. The device according to claim 13, wherein the operation further comprising:

performing circular fit on the range FFT result to obtain a fitting result, which includes the position of the center of the circle;
determining the DC offset based on the fitting result;
calibrating the range FFT result based on the DC offset to obtain a calibrated range FFT result;
selecting data points corresponding to the target range-bin from the range FFT result as the information to be processed, comprising:
selecting data points corresponding to the target range-bin from the calibrated range FFT result as the information to be processed.

15. The device according to claim 13, wherein the operation further comprising: identifying the pelvic floor muscle movement pattern based on the pelvic floor muscle displacement information.

16. The device according to claim 15, wherein the operation further comprising:

performing mean filtering on the pelvic floor muscle displacement information to obtain mean-filtered pelvic floor muscle displacement information;

performing Kalman filtering on the mean-filtered pelvic floor muscle displacement information to obtain Kalman-filtered pelvic floor muscle displacement information;

wherein identifying the pelvic floor muscle movement pattern based on the pelvic floor muscle displacement information, comprising:

identifying the pelvic floor muscle movement pattern based on the Kalman-filtered pelvic floor muscle displacement information.

17. The device according to claim 15, wherein the pelvic floor muscle displacement information includes displacement at multiple time points, and the pelvic floor muscle movement pattern includes contraction, relaxation, contracting, and relaxing;

wherein identifying the pelvic floor muscle movement pattern based on the pelvic floor muscle displacement information, comprising:

calculating the displacement mean and displacement standard deviation corresponding to each time point based on the pelvic floor muscle displacement information according to a predefined sliding window length;

determining the pelvic floor muscle movement pattern corresponding to each time point based on the displacement mean and displacement standard deviation corresponding to each time point.

18. The device according to claim 15, wherein the operation further comprising: real-time displaying the pelvic floor muscle displacement information and/or the identified pelvic floor muscle movement pattern.

19. A computer-readable storage medium, wherein the computer storage medium stores a computer program, and when the computer program is executed on a computer, the computer performs the following operations:

acquiring an intermediate frequency signal;

performing range fast Fourier transform (FFT) on the intermediate frequency signal to obtain a range FFT result, wherein the range FFT result includes multiple sets of data points corresponding to multiple range-bins;

selecting data points corresponding to target range-bin from the range FFT result as information to be processed, wherein the target range-bin matches the range between the pelvic floor muscles and the millimeter-wave radar;

demodulating the information to be processed to obtain phase information;

converting the phase information into pelvic floor muscle displacement information.

20. The storage medium according to claim 19, wherein the operation further comprising:

performing circular fit on the range FFT result to obtain a fitting result, which includes the position of the center of the circle;

determining DC offset based on the fitting result;

calibrating the range FFT result based on the DC offset to obtain a calibrated range FFT result;

selecting data points corresponding to the target range-bin from the range FFT result as the information to be processed, comprising:

selecting the data points corresponding to the target range-bin from the calibrated range FFT result as the information to be processed.

21. The storage medium according to claim 19, wherein the operation further comprising: identifying the pelvic floor muscle movement pattern based on the pelvic floor muscle displacement information.

22. The storage medium according to claim 21, wherein the operation further comprising:

performing mean filtering on the pelvic floor muscle displacement information to obtain the mean-filtered pelvic floor muscle displacement information;

performing Kalman filtering on the mean-filtered pelvic floor muscle displacement information to obtain Kalman-filtered pelvic floor muscle displacement information;

wherein identifying the pelvic floor muscle movement pattern based on the pelvic floor muscle displacement information, comprising:

identifying the pelvic floor muscle movement pattern based on the Kalman-filtered pelvic floor muscle displacement information.

23. The storage medium according to claim 22, wherein the pelvic floor muscle displacement information includes displacement at multiple time points, and the pelvic floor muscle movement pattern includes contraction, relaxation, contracting, and relaxing;

    wherein identifying the pelvic floor muscle movement pattern based on the pelvic floor muscle displacement information, comprising:

    calculating the displacement mean and displacement standard deviation corresponding to each time point based on the pelvic floor muscle displacement information according to a predefined sliding window length;
    determining the pelvic floor muscle movement pattern corresponding to each time point based on the displacement mean and displacement standard deviation corresponding to each time point.

24. The storage medium according to claim 22, wherein the operation further comprising: real-time displaying the pelvic floor muscle displacement information and/or the identified pelvic floor muscle movement pattern.

**100**

Acquisition module
**110**

FFT module
**120**

Calibration module
**130**

Selection module
**140**

Demodulation module
**150**

Conversion module
**160**

Filtering module
**170**

Identification module
**180**

Display module
**190**

**FIG.1**

<u>200</u>

acquiring an intermediate frequency signal
<u>210</u>

performing range FFT on the intermediate frequency signal to obtain a range FFT result
<u>220</u>

selecting data points corresponding to target range-bin from the range FFT result as the information to be processed
<u>240</u>

demodulating the information to be processed to obtain phase information
<u>250</u>

converting the phase information into pelvic floor muscle displacement information
<u>260</u>

identifying pelvic floor muscle movement pattern based on the pelvic floor muscle displacement information
<u>280</u>

real-time displaying the pelvic floor muscle displacement information and/or the identified pelvic floor muscle movement pattern
<u>290</u>

**FIG.2**

**FIG.3**

```
┌─────────────────────────┐
│      intermediate       │
│    frequency signal     │
└─────────────────────────┘
             ⇩
┌─────────────────────────┐
│      A/D sampling       │
└─────────────────────────┘
             │
             ▼
┌─────────────────────────┐
│       range FFT         │
└─────────────────────────┘
             │
             ▼
┌─────────────────────────┐
│       calibration       │
└─────────────────────────┘
             │
             ▼
┌─────────────────────────┐
│    rang-bin selection   │
└─────────────────────────┘
             │
             ▼
┌─────────────────────────┐
│   phase demodulation    │
└─────────────────────────┘
             │
             ▼
┌─────────────────────────┐
│        filtering        │
└─────────────────────────┘
             │
             ▼
┌─────────────────────────┐
│  pattern identification │
└─────────────────────────┘
             │
             ▼
┌─────────────────────────┐
│    real-time display    │
└─────────────────────────┘
```

**FIG.4**

**FIG.5**

## INTERNATIONAL SEARCH REPORT

| International application No. |
| --- |
| **PCT/CN2024/074075** |

### A. CLASSIFICATION OF SUBJECT MATTER

A61B5/05(2021.01)i; A61B5/103(2006.01)i

According to International Patent Classification (IPC) or to both national classification and IPC

### B. FIELDS SEARCHED

Minimum documentation searched (classification system followed by classification symbols)

IPC :A61B5, G01S

Documentation searched other than minimum documentation to the extent that such documents are included in the fields searched

Electronic data base consulted during the international search (name of data base and, where practicable, search terms used)

CNABS: CNTXT; CNKI; VEN: 毫米波, 雷达, FMCW, 盆底, 盆腔, 骨盆, 膀胱, 尿道, 尿失禁, 距离, 距离门, FFT, 傅里叶, 中频, millemeter wave, mmwave, radar, pelvic, urine, bladder, urinary, urethral, range, distance, fourier

### C. DOCUMENTS CONSIDERED TO BE RELEVANT

| Category* | Citation of document, with indication, where appropriate, of the relevant passages | Relevant to claim No. |
| --- | --- | --- |
| A | CN 114533004 A (SHANDONG NORMAL UNIVERSITY) 27 May 2022 (2022-05-27) entire document | 1-24 |
| A | CN 113854981 A (NANJING LIUJI OPTOELECTRONIC TECHNOLOGY RESEARCH INSTITUTE CO., LTD. et al.) 31 December 2021 (2021-12-31) entire document | 1-24 |
| A | CN 103505204 A (NANJING MEDLANDER MEDICAL SCIENCE & TECHNOLOGY CO., LTD.) 15 January 2014 (2014-01-15) entire document | 1-24 |
| A | CN 110045366 A (INFINEON TECHNOLOGIES AG) 23 July 2019 (2019-07-23) entire document | 1-24 |
| A | CN 113286545 A (RENOVIA INC.) 20 August 2021 (2021-08-20) entire document | 1-24 |
| A | US 2011077500 A1 (SHAKIBA, K.) 31 March 2011 (2011-03-31) entire document | 1-24 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **08 April 2024** | **12 April 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/074075**

### C. DOCUMENTS CONSIDERED TO BE RELEVANT

| Category* | Citation of document, with indication, where appropriate, of the relevant passages | Relevant to claim No. |
|---|---|---|
| A | QI, Yingnan et al. "Estimation of Urine Flow Velocity Using Millimeter-Wave FMCW Radar" *Sensors*, Vol. 22, No. 23, 02 December 2022 (2022-12-02), 8402 entire document | 1-24 |

Form PCT/ISA/210 (second sheet) (July 2022)

**INTERNATIONAL SEARCH REPORT**
Information on patent family members

International application No.

**PCT/CN2024/074075**

| Patent document cited in search report | | | Publication date (day/month/year) | Patent family member(s) | | | Publication date (day/month/year) |
|---|---|---|---|---|---|---|---|
| CN | 114533004 | A | 27 May 2022 | None | | | |
| CN | 113854981 | A | 31 December 2021 | None | | | |
| CN | 103505204 | A | 15 January 2014 | None | | | |
| CN | 110045366 | A | 23 July 2019 | US | 2019219687 | A1 | 18 July 2019 |
| | | | | US | 11346936 | B2 | 31 May 2022 |
| | | | | EP | 3511738 | A2 | 17 July 2019 |
| | | | | EP | 3511738 | B1 | 09 March 2022 |
| CN | 113286545 | A | 20 August 2021 | AU | 2019373154 | A1 | 17 June 2021 |
| | | | | CA | 3118295 | A1 | 07 May 2020 |
| | | | | JP | 2022506203 | A | 17 January 2022 |
| | | | | EP | 3873344 | A1 | 08 September 2021 |
| | | | | US | 2021353195 | A1 | 18 November 2021 |
| | | | | WO | 2020092343 | A1 | 07 May 2020 |
| US | 2011077500 | A1 | 31 March 2011 | US | 8460217 | B2 | 11 June 2013 |
| | | | | US | 2013274588 | A1 | 17 October 2013 |
| | | | | US | 9700236 | B2 | 11 July 2017 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310253897